(19) Europäisches Patentamt
European Patent Office
Office européen des brevets

(11) Veröffentlichungsnummer: **0 189 042**
**A2**

(12) # EUROPÄISCHE PATENTANMELDUNG

(21) Anmeldenummer: **86100088.3**

(22) Anmeldetag: **07.01.86**

(51) Int. Cl.⁴: **C 07 C 135/02**, E 21 B 43/22

(30) Priorität: **19.01.85 DE 3501639**

(43) Veröffentlichungstag der Anmeldung: **30.07.86**
**Patentblatt 86/31**

(84) Benannte Vertragsstaaten: **BE CH DE FR GB IT LI NL SE**

(71) Anmelder: **HOECHST AKTIENGESELLSCHAFT,**
**Postfach 80 03 20, D-6230 Frankfurt am Main 80 (DE)**

(72) Erfinder: **Wester, Norbert, Dr., Sachsenring 12,**
**D-6238 Hofheim am Taunus (DE)**
Erfinder: **Schneider, Gerhart, Dr., Ziegelheck 3,**
**D-6240 Königstein/Taunus (DE)**

(54) **Etheraminoxide, Verfahren zu deren Herstellung und deren Verwendung als Tenside für die tertiäre Erdölgewinnung.**

(57) Etheraminoxide der Formel

$$
\begin{array}{c} R_1 \\ | \\ RO-(R_3O)_x-N \rightarrow O \\ | \\ R_2 \end{array}
$$

wobei R $C_8$–$C_{20}$-Alkyl, $C_4$–$C_{20}$-Alkenyl oder $C_4$–$C_{18}$-Alkylaryl $R_1$ und $R_2$ gleich oder verschieden sind und $C_1$–$C_4$-Alkyl, x eine Zahl von 2 bis 12 und $R_3$ eine Gruppe der Formeln $-C_2H_4-$ und/oder $-C_3H_6-$ bedeuten. Diese Aminoxide werden hergestellt durch Oxidation der zugrundeligenden Etheramine der Formel $RO-(R_3O)_x-NR_1R_2$. Die Verbindungen eignen sich als oberflächenaktive Hilfsmittel bei der Erdölförderung.

EP 0 189 042 A2

Etheraminoxide, Verfahren zu deren Herstellung und deren Verwendung als Tenside für die tertiäre Erdölgewinnung

Bei der Gewinnung von Öl aus unterirdischen Lagerstätten gelingt es im allgemeinen nur 20 - 30 % des ursprünglich vorhandenen Öls durch primäre Gewinnungsverfahren zu fördern. Hierbei gelangt das Öl mit Hilfe des natürlichen Lagerstättendrucks an die Erdoberfläche. Bei der sekundären Gewinnung wird Wasser in die geologische Formation eingepreßt und das Öl durch mehrere Produktionssonden gefördert. Dieses Wasserfluten als Sekundärmaßnahme ist relativ billig und wird daher häufig verwendet, führt jedoch in vielen Fällen nur zu einer geringen Mehrentölung der Lagerstätte.

Nach Beendigung der sekundären Erdölfördermaßnahmen ist allein durch Zufuhr mechanischer Energie keine weitere wirtschaftliche Erdölgewinnung zu erreichen. In dem heterogenen Porenraum überholt das niedriger viskose Wasser das höher viskose Öl, so daß fast nur noch Wasser und kein Öl mehr gefördert wird. Hat der Verwässerungsgrad bei ca. 98 % die Wirtschaftlichkeitsgrenze überschritten, so kommen nur noch Verfahren der tertiären Erdölgewinnung in Frage. Darunter versteht man Verfahren, bei denen entweder die Viskosität des Öls erniedrigt und/oder die Grenzflächenspannung zwischen Wasser und Öl erniedrigt wird.

Die meisten Verfahren lassen sich als termische Ölgewinnungsverfahren, Lösungs- oder Mischfluten, Tensid- oder Polymerfluten bzw. als Kombination von mehreren der genannten Verfahren einordnen.

Tensidflutverfahren beruhen auf einer starken Erniedrigung der Grenzflächenspannung zwischen Öl und Flutwasser. Je nach Tensidkonzentration unterscheidet man Tensidfluten (Low tension flooding), micellares Fluten und Emulsionsfluten.

In der Monografie von D.O. Shah und R.S. Schechter: Improved Oil Recovery by Surfactant and Polymerflooding, Academic Press Inc., sowie in zahlreichen Patentschriften werden eine Vielzahl von Tensiden aufgeführt, die beim Tensidflutprozeß Verwendung finden können. Als Tenside werden dabei vor allem Sulfonate, z.B. synthetische und natürliche Petrolsulfonate, Alkylsulfonate, z.B. $C_{13}$-$C_{20}$ sec. Alkansulfonat-Na MG 328/350, α-Olefinsulfonat-Na, Alkylarylsulfonate z.B. Dodecylbenzolsulfonat-Na, Alkyltoluolsulfonate oder Alkylxylolsulfonate beschrieben.

Diese Sulfonate besitzen aber nur eine sehr niedrige Toleranzgrenze gegenüber dem Salzgehalt der Lagerstättengewässer. So sind z.B. Petrolsulfonate nur in einem Wasser mit einem Salzgehalt von 1,5 % NaCl löslich. Sulfonate sind vor allem auch gegen die im Lagerstättenwasser enthaltenen Erdalkalien sehr empfindlich. Bei höheren Salzkonzentrationen bilden sich beim Einsatz dieser Tenside Fällungsprodukte, die zur Verstopfung des porösen Raumes der Formation führen können.

In den Patentschriften DE 3025 383 und DE 3014 510 wird die Verwendung von Aminoxiden, z.B. Alkyldimethylaminoxid oder Naphthenoylamidopropylen-dimethylaminoxid vorgeschlagen. Diese Verbindungen zeichnen sich durch eine Verträglichkeit mit hochsalinem Wasser aus, aber die ölmobilisierende Wirkung ist nicht ausreichend.

Überraschenderweise wurde gefunden, daß Alkyl- und Alkylaryletheraminoxide bei der Verwendung als Tenside für die Erdölförderung den oben beschriebenen Verbindungen überlegen sind.

Gegenstand der Erfindung sind Alkyl- und Alkylaryl-alkoxyaminoxide der Formel:

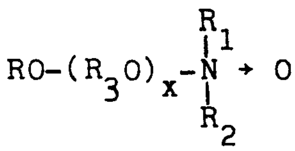

wobei R $C_8$-$C_{20}$-Alkyl, $C_8$-$C_{20}$-Alkenyl oder $C_4$-$C_{18}$-Alkylaryl $R_1$ und $R_2$ gleich oder verschieden sind und $C_1$-$C_4$-Alkyl, x eine Zahl von 2 bis 12 und $R_3$ eine Gruppe der Formeln $-C_2H_4-$ und/oder $-C_3H_6-$ bedeuten.

Als $R_3$ ist Äthylen bevorzugt. Als Alkylarylgruppen sind Alkylphenyl-Gruppen bevorzugt, beispielsweise Octylphenyl, Dinonylphenyl, Dodecylphenyl oder Tributylphenyl. Als Alkylreste sind von Vorteil $C_8$-$C_{18}$, die sowohl aus nativen oder synthetischen Alkoholen (sec. Alkoholen, Oxalkoholen) hergeleitet sein können. Der Begriff Alkylaryl umfaßt Mono-, Di- und Trialkyl-Gruppen, wobei jede Alkylgruppe 4 bis 18, vorzugsweise 4 bis 9, C-Atome haben kann.

Zur Darstellung der erfindungsgemäßen Aminoxide werden die entsprechenden oxethylierten Alkohole bzw. Phenole der allgemeinen Formel

$$RO-(R_3O)_x-H$$

worin R, $R_3$ und x die obengenannten Bedeutungen haben, endständig halogeniert, z.B. mit Thionylchlorid oder $PBr_3$ oder sulfatiert, z.B. mit $SO_3$ oder $Cl-SO_3H$. Diese Zwischenverbindung wird dann im Autoklaven unter Druck bei Temperaturen von ca. 120 bis 200°C, vorzugsweise bei 170°C mit einem Dialkylamin der Formel $HNR_1R_2$ umgesetzt. Die so erhaltenen Etheramine werden dann mit einem geeigneten Oxidationsmittel, beispielsweise mit Wasserstoffperoxid bei Temperaturen von ca. 65 bis 80°C zu den erfindungsgemäßen Aminoxiden oxidiert.

Die Etheraminoxide der genannten Formel sind Tenside, die sich durch Beständigkeit gegenüber Ca-ionen in einem weiten

Temperaturbereich auszeichnen. Sie eignen sich besonders bei der tertiären Erdölförderung, bei der Sondenstimulierung und Frac-Behandlung von Erdöl-Lagerstätten. Der Einsatz der Verbindungen gemäß der vorliegenden Erfindung reduziert den Einpreßdruck (Injektionsdruck) des Flutmediums und erhöht bei diesen Verfahren die Ölausbeute. In allen Fällen wird das Tensid dem Flutwasser im allgemeinen in Mengen von 0,01 bis 10 %, vorzugsweise 0,05 bis 3 % zugesetzt.

Die Etheraminoxide können auch in Kombination mit anionischen Tensiden, wie z.B. Alkansulfonaten, α-Olefinsulfonaten, Petrolsulfonaten, Alkylarylsulfonaten, Alkylxylolsulfonaten und anderen, nichtionischen Tensiden wie Alkyl- bzw. Alkylphenolpolyglykolethern eingesetzt werden. Von besonderem Interesse sind dabei Mischungen aus den erfindungsgemäßen Aminoxiden und Alkansulfonaten in Mischungsverhältnissen von 1 : 1 bis 1 : 4. Als weitere Zusätze kommen Alkohole und Glykolether in Frage. Die Viskosität des Flutwassers kann außerdem durch Polymere, wie z.B. Hydroxiethylcellulose, Polyacrylamide, Copolymere auf Basis Acrylamid oder Polysaccharide erhöht werden.

Beispiel 1

Herstellung von Lauryldioxethyldimethylaminoxid

a) 464 g (0,82 Mol) Lauryldioxethylsulfat-Na (68 %), 139,4 g (1,24 Mol) Dimethylamin-Lösung (40 %), 40 g (1,0 Mol) - NaOH werden unter Rühren zusammen mit 700 ml dest. Wasser 10 Std. bei 175°C in einem Autoklaven erhitzt. Nach Abdestillieren des restlichen Wassers und des überschüssigen Dimethylamins aus der organischen Phase erhält man 245 g (252 g d. Th.) des tertiären Amins (ges. bas. N: 4,2 % / tert. N: 4,2 %).

b) 100 g (0,31 Mol) des Amins werden in 210 ml Wasser emulgiert und auf 70°C erhitzt. Ohne weitere Erwärmung tropft man 37,1 g (0,33 Mol) $H_2O_2$ (30 %) und rührt 4 Stunden

bei 70°C nach. Dabei entsteht das Lauryldioxethyldimethylaminoxid in nahezu quantitativer Ausbeute.

Beispiel 2

Herstellung von Tributylphenoloctaoxethyldimethylaminoxid

Entsprechend dem in Beispiel 1 beschriebenen Verfahren wird Tributylphenyloctaoxethylsulfat mit Dimethylamin zum Amin umgesetzt. (tert. N: 1,8 % / ges. bas. N: 1,8 %) und mit $H_2O_2$ zum Aminoxid oxidiert.

Beispiel 3

Herstellung von Cocosalkylpentaoxethyldimethylaminoxid

Analog dem in Beispiel 1 angegebenen Verfahren wird Cocosalkylpentaoxethylchlorid mit Dimethylamin zum tertiären Amin (tert. N: 2,6 %) ges. bas. N: 2,6 %) und mit $H_2O_2$ zum Dimethylaminoxid umgesetzt.

Zur Bestimmung der Wirksamkeit der erfindungsgemäßen Verbindungen wird die in der US Patentschrift 4008 165 beschriebene Mikrokapillarentölungs-Methode, die Bestimmung der Grenzflächenspannung nach der Spinning-drop-Tensiometer-Methode und das Phasenverhalten nach W i n s o r herangezogen.

Bei der Mikrokapillarentölung werden als Modell für den Porenraum der Lagerstätte Mikrokapillaren aus Glas der Fa. Drummond Scientific Co / USA verwendet, die bei einem Volumen von 5 µl eine Länge von 30 mm und einen Durchmesser von 0,45 mm aufwiesen.
Die Mikrokapillaren werden an einem Ende abgeschmolzen, in einem Exsikator evakuiert und mit Rohöl gefüllt
Die Kapillaren werden in Tensidlösungen (Reagenzgläser), die im Wassebad temperiert werden, mit der Öffnung nach oben senkrecht eingebracht und die Verdrängung des Öls wird in Abhängigkeit von der Zeit visuell registriert.

Die Versuche werden 10mal wiederholt und der Mittelwert gebildet.

Mit Hilfe des folgenden Beurteilungsschemas kann die Wirksamkeit der Tenside in Abhängigkeit von deren Konzentration, der Salzkonzentration, pH-Wert, Temperatur und Ölzusammensetzung bestimmt werden.

| <u>Wert</u> | | | | | |
|---|---|---|---|---|---|
| 9 | leer (30 mm) nach 10 Minuten | | | | |
| 8 | leer nach 1 Stunde | | | | |
| 7 | leer nach 3 Stunden | | | | |
| 6 | leer nach 20 Stunden | | | | |
| 5 | 16-25 mm | Entleerung | nach | 20 | Stunden |
| 4 | 9-15 mm | " | " | 20 | " |
| 3 | 4-8 mm | " | " | 20 | " |
| 2 | 1-3 mm | " | " | 20 | " |
| 1 | Spur | " | " | 20 | " |
| 0 | unverändert | | " | 20 | " |

Diese Methode bietet den Vorteil, daß bei dem geringen Durchmesser der Mikrokapillaren Viskosität und Dichte der Öle keinen großen Einfluß auf die Entölungswirkung ausüben und es möglich ist, mit Lagerstättenöl und Lagerstättenwasser zu arbeiten.

Nach Taber J. Petr. Techn. 3 (1969), S. 3 - 12 sind Tenside für die tertiäre Erdölgewinnung nur geeignet, wenn die Grenzflächenspannung an der Phasengrenze Öl/Salzwasser auf Werte kleiner $10^{-2}$ $mNm^{-1}$ erniedrigt wird. Für diese Bestimmung der Grenzflächenspannung an der Phasengrenze Öl/Wasser wird das von Wade und Burkowsky entwickelte Spinning-drop-Interfacial-Tensiometer verwendet. (M. Burkowsky and C. Marx: Über den Mechanismus des Tensidflutens in hochsalinaren Systemen; Erdöl-Erdgas-Zeitschrift 95 (1979) S. 17 - 25.

Die Methode beruht darauf, daß ein Öltropfen, der in eine um die horizontale Achse rotierende Kapillare gebracht wird, die eine Flüssigkeit (Salzwasser und Tensid) mit höherer Dichte enthält, defomiert wird. Der Tropfen wird gestreckt, bis ein Gleichgewicht der deformierenden Kräfte und der Grenzflächenspannung erreicht wird.

Die Grenzflächenspannung errechnet sich nach Vonnegut (B. Vonnegut, Rev. Sci. Instruments 13 (1942), S. 6 - 9) und Princen (H.M. Princen, J.Y.Z. ZIA und S.G. Mason, J. Coll. Int. Sci 23, (1967) 99 - 107)

Aus dem gemessenen Öltropfendurchmesser R, der Rotationsgeschwindigkeit W und dem Dichteunterschied Δd nach folgender Formel:

$$\gamma\ 1/2 = \frac{\Delta d \cdot W^2 \cdot R^3}{4} \quad (mNm^{-1})$$

Die mit diesen Methoden gemessenen Werte sind in den folgenden Tabellen zusammengefaßt. Es wurden in allen Fällen jeweils 1 %ige wäßrige Lösungen der Tenside verwendet. Als Vergleichsprodukt wurde gemäß dem Stand der Technik Cocosdimethylaminoxid verwendet.
Nach dem heutigen stand der Aufklärung des Mechanismus der Entölung beim Tensidfluten ist die Ausbildung einer 3. Phase (Mittelphase) einer Mikroemulsion die Voraussetzung für ein optimales Tensidflutergebnis.
[Rieckmann, M. Tertiäre Erdölgewinnung, Erdöl und Kohle-Erdgas-Petrochemie 36 (1983) 281 - 282) Healy, R.N. und Reed, R.L. Soc. Petr. Eng. J. 10 (1979) 492 - 501), Obah, B. und Neumann, H.J. Über die Bildung von Mikroemulsion Tenside Detergents 20 (1983) 145 - 151)]

Diese gesuchte dritte Phase entsteht im System, wenn die Grenzflächenspannung an der Phasengrenze Öl/Salzwasser stark erniedrigt wird.

Bei der Ermittlung der Mittelphasen werden 5 ml Tensidlösung (mit Salzwasser) und 5 ml Öl in ein Reagenzglas gebracht, das Reagenzglas zugeschmolzen, kräftig geschüttelt und in einem Trockenschrank bei konstanter, gewählter Temperatur gelagert.

Nach einer Stunde Lagerzeit wird erneut kräftig geschüttelt und die Reagenzgläser werden dann ohne weitere Durchmischung gelagert. Nach einer Lagerzeit von 1 Tag und 7 Tagen wird die Bildung einer Mittelphase ermittelt.

Versuch I

| | Grenzflächenspannung $mNm^{-1}$ | | Mikrokapillarentölung | | Mittelphasenbildung | |
|---|---|---|---|---|---|---|
| Lagerstättenöle (1) und (2) mit 200 g NaCl/l 40°C, 1 % WS | (1) | (2) | (1) | (2) | (1) | (2) |
| $Lauryl\text{-}(OCH_2CH_2)_2\text{-}\overset{O}{\overset{\uparrow}{N}}(CH_3)_2$ | $1x10^{-2}$ | $2x10^{-2}$ | 9 | 8 | + | + |
| Cocosdimethylaminoxid | $4x10^{-1}$ | $3x10^{-1}$ | 0 | 0 | – | – |

Versuch II

| | | | | | | |
|---|---|---|---|---|---|---|
| Lagerstättenöle (1) und (2) mit 180 g NaCl/20 g $CaCl_2$/l 40°C, 1 % WS | (1) | (2) | (1) | (2) | (1) | (2) |
| $Lauryl\text{-}(OCH_2CH_2)_2\text{-}\overset{O}{\overset{\uparrow}{N}}(CH_3)_2$ | $8x10^{-3}$ | $1x10^{-2}$ | 9 | 9 | + | + |
| $Cocos\text{-}Alkyl\text{-}\overset{CH_3}{\underset{CH_3}{N}} \rightarrow O$ | $1x10^{-1}$ | $3x10^{-1}$ | 0 | 0 | – | – |

Versuch III

| | | | | | | |
|---|---|---|---|---|---|---|
| Lagerstättenöl B 200 g NaCl/l 40/60°C, 1 % WS | 40°C | 60°C | 40°C | 60°C | 40°C | 60°C |
| $Lauryl\text{-}(OCH_2CH_2)_2\text{-}\underset{O}{\underset{\downarrow}{N}}(CH_3)_2$ | $6x10^{-3}$ | $7x10^{-3}$ | 9 | 9 | + | + |
| $Cocos\text{-}Alkyl\text{-}\overset{CH_3}{\underset{CH_3}{N}} \rightarrow O$ | $2x10^{-1}$ | $5x10^{-1}$ | 0 | 0 | – | – |

Versuch IV

| | Grenzflächenspannung $mNm^{-1}$ | | Mikrokapillarentölung | | Mittelphasenbildung | |
|---|---|---|---|---|---|---|
| Lagerstättenöl K 140 g NaCl/60 g $CaCl_2$/l | 25°C | 40°C | 25°C | 40°C | .25°C | 40°C |
| Lauryl-$(OCH_2CH_2)_2$-$N(CH_3)_2$<br>↓<br>O | $1x10^{-2}$ | $1x10^{-2}$ | 8 | 8 | + | + |
| $CH_3$<br>Cocos-(Alkyl)-N → O<br>$CH_3$ | $5x10^{-1}$ | $2x10^{-1}$ | 0 | 0 | - | - |

Versuch V

| | | | | | | |
|---|---|---|---|---|---|---|
| Aromat. Öl 180 g NaCl/20 g $CaCl_2$/l 60 80°C, 1 % WS | 60°C | 80°C | 60°C | 80°C | 60°C | 80°C |
| Tributylphenyl-<br>$(OCH_2$-$CH_2)_{7,5}$-$N(CH_3)_2$<br>↓<br>O | $1x10^{-2}$ | $1x10^{-2}$ | 6 | 8 | + | + |
| $CH_3$<br>Cocos-Alkyl-N → O<br>$CH_3$ | $8x10^{-1}$ | $2x10^{-1}$ | 0 | 0 | - | - |

Versuch VI

| | | | | | | |
|---|---|---|---|---|---|---|
| Lagerstättenöl K (1)<br>Naphten. Öl (2)<br>80°C,1 % WS | (1) | (2) | (1) | (2) | (1) | (2) |
| Tributylphenyl-<br>$(OCH_2CH_2)_{7,5}$-$N(CH_3)_2$<br>↓<br>O | $6x10^{-3}$ | $1x10^{-2}$ | 9 | 8 | + | + |
| $CH_3$<br>Cocos-(Alkyl)-N → O<br>$CH_3$ | $4x10^{-1}$ | $8x10^{-1}$ | 0 | 0 | - | - |

Versuch VII

| | | | | | | |
|---|---|---|---|---|---|---|
| Naphten. Öl 140 g NaCl/60 g $CaCl_2$/l 80°C, 1 % WS | | | | | | |
| Tributylphenyl-<br>$(OCH_2CH_2)_{7,5}$-$N(CH_3)_2$<br>↓<br>O | $2x10^{-2}$ | | 8 | | + | |
| Cocos-(Alkyl)-$N(CH_3)_2$<br>↓<br>O | $4x10^{-1}$ | | 0 | | - | |

Versuch VIII

Lagerstättenöle K, 200 g NaCl/1 bzw. (1)
180 g NaCl/20 g $CaCl_2$/1 (2)

60°C, 1 % WS, pH 6,5

| Isotridecyl-$O(CH_2CH_2O)_5-N(CH_3)_2$ (↓O) | + sec-Alkansulfonat Na ($C_{13}$-$C_{18}$/MG 328) | Grenzflächensp. ($mNm^{-1}$) (1) | (2) | Mikrokapillar-entölung (1) | (2) | Mittelphasen-bildung (1) | (2) |
|---|---|---|---|---|---|---|---|
| 0,2 % | 0,8 % | $1x10^{-2}$ | $5x10^{-2}$ | 9 | 2 | + | - |
| 0,33 % | 0,66 | $9x10^{-3}$ | $1x10^{-2}$ | 9 | 9 | + | + |
| 0,5 % | 0,5 % | $4x10^{-3}$ | $5x10^{-3}$ | 9 | 8 | + | + |

Versuch IX

Lagerstättenöle K, 200 g NaCl/1 bzw. (1)
45 g NaCl/5 g $CaCl_2$/1 (2)

60°C, 1 % WS

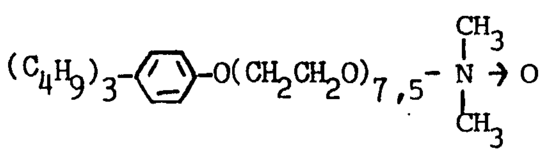

| | + sec-Alkanonsulfonat Na | (1) | (2) | (1) | (2) | (1) | (2) |
|---|---|---|---|---|---|---|---|
| 0,2 % | 0,8 % | $1x10^{-2}$ | $5x10^{-2}$ | 9 | 0 | + | - |
| 0,33 % | 0,66 | $1x10^{-2}$ | $1x10^{-2}$ | 9 | 8 | + | + |
| 0,50 % | 0,50 % | $1x10^{-2}$ | $1x10^{-2}$ | 8 | 9 | + | + |

Versuch X

Lagerstättenöle K, 200 g NaCl/l bzw. (1)
180 g NaCl/20 g $CaCl_2$/l (2)

60°C, 1 % WS, pH 6,5

| Cocos-Alkyl-$(OCH_2CH_2)_5-N(CH_3)_2$ ($\downarrow$ O) | + sec-Alkansulfonat Na ($C_{13}-C_{18}$/MG 328) | Grenzflächensp. ($mNm^{-1}$) (1) | (2) | Mikrokapillar-entölung (1) | (2) | Mittelphasen-bildung (1) | (2) |
|---|---|---|---|---|---|---|---|
| 0,2 % | 0,8 % | $9x10^{-3}$ | $3x10^{-2}$ | 9 | 2 | + | – |
| 0,33 % | 0,66 % | $3x10^{-3}$ | $1x10^{-2}$ | 8 | 5 | + | + |
| 0,50 % | 0,50 % | $2x10^{-2}$ | $9x10^{-3}$ | 8 | 8 | + | + |

Versuch XI

Lagerstättenöle K, 200 g NaCl/l bzw. (1)
160 g NaCl/40 g $CaCl_2$/l (2)

60°C, 1 % WS, pH 6,5

| Cocos-Alkyl-$(OCH_2CH_2)_8-N(CH_3)_2$ ($\downarrow$ O) | + sec-Alkansulfonat Na ($C_{13}-C_{18}$/MG 328) | Grenzflächensp. ($mNm^{-1}$) (1) | (2) | Mikrokapillar-entölung (1) | (2) | Mittelphasen-bildung (1) | (2) |
|---|---|---|---|---|---|---|---|
| 0,2 % | 0,8 % | $1x10^{-3}$ | $5x10^{-2}$ | 9 | 0 | + | – |
| 0,33 % | 0,66 % | $1x10^{-3}$ | $1x10^{-2}$ | 9 | 8 | + | + |
| 0,50 % | 0,50 % | $1x10^{-2}$ | $2x10^{-3}$ | 9 | 8 | + | + |

Die Versuchsergebnisse (Tabellen I - XI) zeigen, daß die Etheraminoxide und deren Mischungen mit sec-Alkansulfonaten ($C_{13}$-$C_{18}$, MG 328) bei unterschiedlichen Salzgehalten (50 - 200 g/l), Temperaturen (20 - 80°C) und auch bei hohen Gehalten an Ca-Ionen den bereits bekannten Alkyldimethylaminoxiden als Tenside für die tertiäre Erdölgewinnung überlegen sind.

0 189 042

Patentansprüche:

HOE 85/F 009

1. Etheraminoxide der Formel

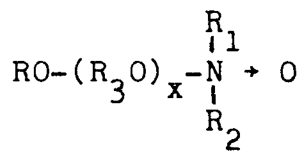

wobei R $C_8$-$C_{20}$-Alkyl, $C_8$-$C_{20}$-Alkenyl oder $C_4$-$C_{18}$-Alkylaryl $R_1$ und $R_2$ gleich oder verschieden sind und $C_1$-$C_4$-Alkyl,
x eine Zahl von 2 bis 12 und $R_3$ eine Gruppe der Formeln $-C_2H_4-$ und/oder $-C_3H_6-$ bedeuten.

2. Verfahren zur Herstellung der Etheraminoxide nach Anspruch 1, dadurch gekennzeichnet, daß eine Verbindung der Formel

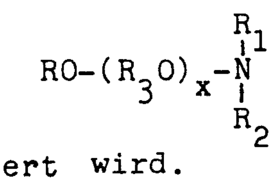

oxidiert wird.

3. Verwendung der Verbindungen nach Anspruch 1 als Hilfsmittel bei der Erdölförderung.

4. Verwendung der Verbindungen 1 zusammen mit Alkansulfonaten, α-Olefinsulfonaten, Dedecylbenzolsulfonaten, Petrolsulfonaten, Alkyl- oder Alkylphenylpolyglykolethern, Alkylarylsulfonaten oder Alkyltoluolsulfonaten.